# EUROPÄISCHE PATENTSCHRIFT

(11) **EP 1 368 361 B1**
(45) Veröffentlichungstag und Bekanntmachung des Hinweises auf die Patenterteilung: **27.04.2005**
(21) Anmeldenummer: 02750508.0
(22) Anmeldetag: 28.02.2002
(51) Int. Cl.: C07F 9/09

(54) **DERIVAT VON PHOSPHORSÄUREESTERSALZ ODER -ADDUKT ODER GEMISCHEN HIERVON, VERFAHREN ZU DEREN HERSTELLUNG UND DEREN VERWENDUNG**
DERIVATIVE OF PHOSPHORIC ACID ESTER SALT OR PHOSPHORIC ACID ESTER ADDUCT OR MIXTURES THEREOF, METHOD FOR THE PRODUCTION THEREOF AND THEIR USE
DERIVE DE SEL D'ESTER D'ACIDE PHOSPHORIQUE OU DE PRODUIT D'ADDITION D'ESTER D'ACIDE PHOSPHORIQUE, OU DE MELANGE DE CES DERNIERS, LEUR PROCEDE DE PRODUCTION ET LEUR UTILISATION

(30) Priorität: 14.03.2001 DE 10112155
(43) Veröffentlichungstag der Anmeldung: 10.12.2003
(73) Patentinhaber: Chemische Fabrik Budenheim KG, 55257 Budenheim (DE)
(72) Erfinder: GÖTZMANN, Karl, 55257 Budenheim (DE); FUTTERER, Thomas, 65197 Wiesbaden (DE); NÄGERL, Hans-Dieter, 67373 Dudenhofen (DE); MANS, Vincent, E-08915 Badalona (ES)
(74) Vertreter: Weber, Dieter, Dr.
(86) Internationale Anmeldenummer: PCT/DE2002/000753
(87) Internationale Veröffentlichungsnummer: WO 2002/072590

(56) Entgegenhaltungen:
- EP-A- 0 116 846
- EP-A- 0 919 591
- WO-A-93/05118

## Beschreibung

Die Verwendung oligomerer Phosphorsäureester als Flammschutzmittel speziell für Polyurethanschäume, ist aus der EP-A-0 658 561 und der DE-A-195 40 861 bekannt. Aus der DE-A-198 27 845 sind flammgeschützte Polyesterformmassen bekannt, die Umsetzungsprodukte von Polyolphosphorsäureester mit wenigstens einer organischen Stickstoffbase, wie Melamin, enthalten. Ein Verfahren zur Herstellung von Polyol-phosphorsäureestern ist aus der WO 99/05200 bekannt. In der Vergangenheit erlangten jedoch Phosphorsäureester als Flammschutzmittel keine große Bedeutung, da ihre Herstellung kompliziert und aufwendig ist und die beispielsweise aus der DE-A-198 27 845 bekannten Phosphorsäureestersalze oder -addukte eine zu hohe Löslichkeit in Wasser besitzen.

Die der Erfindung zugrunde liegende Aufgabe bestand somit darin, neue, als Flammschutzmittel verwendbare Verbindungen auf der Basis von Phosphorsäureestern zu erhalten, die möglichst einfach herstellbar sind und in Wasser wesentlich geringer löslich als die bekannten Phosphorsäureesterderivate sind. Außerdem ist es erwünscht, solche neuen Verbindungen mit besserer thermischer Stabilität zu gewinnen.

Diese Aufgabe wird erfindungsgemäß mit Derivaten von Phosphorsäureestersalzen oder -addukten oder Gemischen hiervon gelöst, welche aus einem Umsetzungsprodukt wenigstens eines Polyol-phosphorsäureesters mit wenigstens einer organischen Stickstoffbase erhalten wurden und die dadurch gekennzeichnet sind, daß dieses Umsetzungsprodukt mit wenigstens einem Aldehyd polykondensiert ist.

Durch die besagte erfindungsgemäße Polykondensation mit Aldehyd bekommt man eine Löslichkeit in Wasser, die nur etwa ein Hundertstel oder weniger derjenigen der entsprechenden nicht polykondensierten Phosphorsäureestersalze oder -addukte beträgt, ohne daß die bekannten Flammschutzeigenschaften leiden. Außerdem besitzen die erfindungsgemäßen Polykondensate erhöhte thermische Stabilität. Insgesamt führen die erhaltenen Eigenschaften dazu, daß die erfindungsgemäßen Derivate mit Vorteil als Flammschutzmittel bzw. intumeszierende Anstrich- und Beschichtungssysteme eingesetzt werden können.

Bei Verwendung als Flammschutzmittel in Kunststofformlingen, speziell für Polyolefinformlinge, wie insbesondere für Polyethylen und Polypropylen, können die erfindungsgemäßen Polykondensate in Pulverform dem aufgeschmolzenen Kunststoff zugesetzt und in ihm, beispielsweise in einem Extruder, gleichmäßig verteilt werden.

Wie erwähnt, können die erfindungsgemäßen Derivate aber auch Anstrich- oder Beschichtungsmitteln zugemischt werden, um Gegenständen unterschiedlichster Beschaffenheit flammhemmende Oberflächeneigenschaften zu verleihen.

Schließlich können die erfindungsgemäßen Derivate auch direkt in einem porösen Träger polykondensiert und in den Poren unlöslich gemacht werden, wie beispielsweise als feuerhemmende Ausrüstung auf Fasern, insbesondere Kunstfasern.

Für die Herstellung des Polykbndensats verwendet man zweckmäßig einen C₁-C₄-Monoaldehyd oder einen C₂-C₄-Dialdehyd, wobei besonders bevorzugt die einfachsten Mono- und Dialdehyde, nämlich Formaldehyd und Acetaldehyd sowie Glyoxal eingesetzt werden.

Die als Ausgangsstoffe erfindungsgemäß eingesetzten Polyol-phosphorsäureester können die aus der Literatur als Flammschutzmittel bekannten oligomeren Phosphorsäureester sein, wie beispielsweise jene, die aus der EP-A-0 658 561 und der DE-A-195 40 861 bekannt sind. Als Polyole dieser Phosphorsäureester werden beispielsweise 1,2-Ethandiol, 1,2- oder 1,3-Propandiol, 1,2-, 1,3-, 1,4-oder 2,3-Butandiol, Glycerin, Trimethylolmethan, -ethan oder -propan, Neopentylglycol, Erythritole, Pentaerythritol, Di- oder Polypentaerythritole, Pentitole, wie Arabitol und Xylitol, Hexitole, wie Manitol oder Sorbitol, Pentositole, Dehydroxybenzole, 2,3-Dimethylol-1,3-dihydroxybenzol oder deren Mischungen verwendet. Bevorzugt sind dabei C₂-C₆-, insbesondere C₄-C₆-Polyole, insbesondere Pentaerythritol oder Sorbitol.

Die Polyol-phosphorsäureester werden mit wenigstens einer organischen Stickstoffbase zu einem Salz oder Addukt umgesetzt. Als organische Stickstoffbase kommen beispielsweise Polyvinylamin, Polyethylenimin, Methylendiamin, Melamin, Guanidin, Methylolmelamin oder deren Kondensate sowie deren Gemische in Betracht. Bevorzugt wird als organische Stickstoffbase Melamin eingesetzt. In dem erfindungsgemäß polykondensierten Umsetzungsprodukt ist das Molverhältnis von Stickstoffbase zu Polyolphosphorsäureester vorzugsweise 2-3 mol Stickstoffbase je 1 mol Polyolphosphorsäureester.

Bezogen auf 1 mol Stickstoffbase, vorzugsweise Melamin, wird das Phosphorsäureestersalz oder - addukt zweckmäßig mit 0,5-8, vorzugsweise 0,5-4, besonders bevorzugt 0,5-2 mol Monoaldehyd bzw. 0,25-4, vorzugsweise 0,25-2, besonders bevorzugt 0,25-1 mol Dialdehyd, wie Glyoxal, polykondensiert.

Durch Auswahl verschiedener Variabler lassen sich die Eigenschaften der erzielten Flammschutzmittel an verschiedenste Einsatzzwecke anpassen. Hierzu gehört die Auswahl der Polyole bei der Esterherstellung, das Molverhältnis von Hydroxylgruppen des Polyols zur verwendeten Menge an Phosphorpentoxid bei der Veresterung, das Verhältnis von Phosphorsäureestergruppen zur Menge an organischer Stickstoffbase bei der Salz- bzw. Adduktbildung sowie die Einsatzmenge an Aldehyd bei der Polykondensation.

Bei Verwendung intermolekularer Diole entstehen Ester, die bei Normalbedingungen noch fließfähig sind, während bei Verwendung höherer Polyole, wie Pentaerythritol, Ester von bitumenartiger Konsistenz entstehen, die nur als konzentrierte wäßrige Lösungen verwendet werden können.

In der Regel wird man versuchen, möglichst alte Hydroxylgruppen des Polyols zu phosphorylieren und dabei je zwei Hydroxylgruppen 1 mol Phosphorpentoxid verwenden. Dabei wird ein möglichst hoher prozentualer Phosphorgehalt angestrebt. Besonders vorteilhaft sind Polyolphosphorsäureester, deren Phosphorgehalt über 20% liegt. Wegen des Phosphorgehalts im Endprodukt wird man bei der Salz- oder Adduktbildung in der Regel mit 1 mol Melamin je 2 mol Phosphorsäureestergruppen auskommen. Besonders vorteilhaft erwies es sich, bei der Umsetzung Phosphorsäureestersalz bzw. -addukt von 1,5-2,5 mol NH₂-Gruppen in der organischen Stickstoffbase je Estergruppe vorzusehen. Zweckmäßig werden je mol Melamin 0,5-2 mol Formaldehyd angewendet.

Das bevorzugte Verfahren zur Herstellung der erfindungsgemäßen Derivate oder Derivatgemische besteht darin, daß man bei der Estergewinnung aus Polyol mit Phosphorpentoxid die erforderliche Menge an Phosphorpentoxid in einer Menge des herzustellenden Esters oder Estergemisches suspendiert und sodann bei einer Temperatur im Bereich von 20-180°C die für die Veresterung erforderliche Menge an Polyol und gegebenenfalls an Wasser zugibt, sodann die Veresterungsreaktion im wesentlichen vollständig ablaufen läßt und dann das Produkt gewinnt. Dabei kann man mit stöchiometrischen oder im wesentlichen stöchiometrischen Mengen an Phosphorpentoxid, Polyol, gegebenenfalls Wasser arbeiten, so daß praktisch nur der oder die gewünschten Ester entstehen und keine nachträgliche Abtrennung von überschüssigen Ausgangsstoffen oder Lösungsmitteln erfolgen muß.

Die Zugabe von Polyol und gegebenenfalls Wasser erfolgt zweckmäßig bei einer Temperatur im Bereich von 20-150°C, die anschließende Vervollständigung der Veresterungsreaktion zweckmäßig während 1-6 Stunden bei wenigstens der Temperatur der Polyolzugabe. Zweckmäßig wird bei der Vervollständigung der Veresterungsreaktion so lange erhitzt und gerührt, bis eine klare Flüssigkeit entstanden ist, die anzeigt, daß die Umsetzung vollständig erfolgte.

Wenn die Reaktion abgeschlossen ist, wird die neu entstandene Menge an Phosphorsäureester oder -gemisch aus dem Reaktionsbehälter entnommen. Darauf kann mit der im Reaktionsbehälter verbliebenen Restmenge an Phosphorsäureester ein neuer Ansatz der Umsetzung begonnen werden, indem erneut Phosphorpentoxid zugegeben und in dem fertigen Ester oder Estergemisch suspendiert wird.

Der so erhaltene Polyol-phosphorsäureester wird mit organischer Stickstoffbase unter Bildung von Salzen oder Addukten umgesetzt. Diese Umsetzung kann getrennt von der Esterbildung mit den abgetrennten Estern oder aber im gleichen Reaktionsgefäß wie die Esterbildung durchgeführt werden.

Anschließend wird durch Zugabe mindestens der stöchiometrischen Menge an Aldehyd, wie insbesondere Formaldehyd, die Polykondensation durchgeführt. Die Temperatur der Polykondensation liegt zweckmäßig im Bereich von 50-100°C. Das durch seine Schwerlöslichkeit in Wasser sich bildende feste Polykondensat wird sodann abfiltriert und in Form eines Pulvers als Flammschutzmittel eingesetzt.

Wenn mit dem Flammschutzmittel Fasern oder andere poröse Trägermaterialien imprägniert werden sollen, ist es zweckmäßig, die Fasern oder anderen porösen Gegenstände mit einer wäßrigen Lösung des Phosphorsäureestersalzes oder -adduktes zu tränken und sodann die getränkte Faser mit dem Aldehyd zu behandeln, wobei sich in den Poren der Faser oder des anderen porösen Trägers das erwünschte Polykondensat bildet.

### Beispiel

In einem mit Rührer, Thermometer, Rückflußkühler und Tropftrichter ausgestatteten Rundkolben von 11 Nutzinhalt werden 500 g Pentaerythrittetraorthophosphorsäuremonoester vorgelegt. Wegen der Zähflüssigkeit dieses Stoffes wird zuerst unter Rühren auf eine Temperatur von 80-90°C erwärmt. Ist diese Temperatur erreicht, werden 142 g Phosphorpentoxid zugegeben und unter Rühren suspendiert. Anschließend wird eine pastenförmige Mischung aus 68 g Pentaerythrit und 18 g Wasser portionsweise eingetragen. Wenn alles zugegeben ist, wird die Temperatur auf 150°C gesteigert, und so lange bei dieser Temperatur gerührt, bis eine klare Flüssigkeit entstanden ist. Nach Abkühlen auf etwa 80°C wird die entstandene Menge Pentaerythrittetraphosphorsäuremonoester (228 g) entnommen.

In eine auf 80°C erwärmte Melaminsuspension wird unter Rühren soviel von der oben erzeugten 80%-igen wäßrigen Esterlösung eingebracht, wie dem gewünschten Molverhältnis von 1 mol Ester je 3 mol Melamin entspricht. Es findet sofort eine erkennbare Reaktion statt, die sich durch eine starke Viskositätserhöhung zeigt. Nach einigen Minuten sinkt die Viskosität wieder ab, womit die Salzbildung beendet ist.

Sodann wird das gebildete Salz mit Formaldehyd polykondensiert. Es erwies sich als günstig, zur Polykondensation 0,5-8 mol Formaldehyd je mol Melamin zu verwenden, bevorzugt 0,5-4 mol Formaldehyd pro mol Melamin und besonders bevorzugt 0,5-2 mol Formaldehyd pro mol Melamin.

Die Formaldehydzugabe erfolgt in Form einer handelsüblichen 37%-igen Lösung. Nach der Zugabe wird die Feststoffsuspension noch 30 Minuten bei 80°C gerührt, und danach wird der Feststoff mit einem geeigneten Filter abgetrennt. Der Filterrückstand wird mit Wasser nachgewaschen und anschließend getrocknet. Filtrat und Waschwasser werden vereinigt und bei einem nächsten Ansatz als Suspensionsmedien für das Melamin eingesetzt.

Das auf diese Weise gebildete erfindungsgemäße Flammschutzmittel hatte folgende Eigenschaften:

| | |
|---|---|
| Beschaffenheit | reines Pulver |
| P-Gehalt | etwa 23% |
| N-Gehalt | etwa 27% |
| pH-Wert (1%-ig) | etwa 3,6 |
| Schüttgewicht | g/l etwa 700 |
| Zersetzungstemperatur | ca.310°C |
| Intumeszenz | stark |
| Wasserlöslichkeit | bei 20°C < 0,02 g/100 ml Wasser |
| | bei 80°C < 0,05 g/100 ml Wasser |

## Patentansprüche

1. Derivat von Phosphorsäureestersalz oder -addukt oder Gemischen hiervon, erhalten aus einem Umsetzungsprodukt wenigstens eines Polyol-phosphorsäureesters mit wenigstens einer organischen Stickstoffbase, **dadurch gekennzeichnet, daß** dieses Umsetzungsprodukt mit wenigstens einem Aldehyd polykondensiert ist.

2. Derivat nach Anspruch 1, **dadurch gekennzeichnet, daß** das Umsetzungsprodukt mit einem C₁-C₄-Monoaldehyd oder einem C₂-C₄-Dialdehyd polykondensiert ist.

3. Derivat nach Anspruch 1 oder 2, **dadurch gekennzeichnet, daß** das Umsetzungsprodukt mit Formaldehyd, Acetaldehyd oder Glyoxal polykondensiert ist.

4. Derivat nach einem der Ansprüche 1 bis 3, **dadurch gekennzeichnet, daß** es mit 0,5-8, vorzugsweise 0,5-4, besonders 0,5-2 mol Monoaldehyd bzw. mit 0,25-4, vorzugsweise 0,25-2, besonders 0,25-1 mol Dialdehyd pro mol organische Stickstoffbase, vorzugsweise Melamin polykondensiert ist.

5. Derivat nach einem der Ansprüche 1 bis 4, **dadurch gekennzeichnet, daß** das Umsetzungsprodukt durch Umsetzung von 2-3 mol organischer Stickstoffbase je mol Polyolphosphorsäureester erhalten wurde.

6. Derivat nach einem der Ansprüche 1 bis 5, **dadurch gekennzeichnet, daß** das Umsetzungsprodukt unter Verwendung von Melamin, Guanidin oder Ethylendiamin, insbesondere Melamin, als Stickstoffbase erhalten wurde.

7. Derivat nach einem der Ansprüche 1 bis 6, **dadurch gekennzeichnet, daß** der Polyolphosphorsäureester unter Verwendung wenigstens eines C₄-C₆-Polyols, vorzugsweise Pentaerythritol oder Sorbitol, erhalten wurde.

8. Verfahren zur Herstellung eines Derivates von Phosphorsäureestersalz oder -addukt oder eines Gemisches hiervon durch Umsetzung wenigstens eines Polyols mit Phosphorpentoxid und Umsetzung des so erhaltenen Polyol-phosphorsäureesters mit wenigstens einer organischen Stickstoffbase unter Bildung eines Phosphorsäureestersalzes oder -adduktes, **dadurch gekennzeichnet, daß** man die für die Veresterung erforderliche Menge an Phosphorpentoxid in einer Menge des herzustellenden Esters oder Estergemisches suspendiert, sodann bei einer Temperatur im Bereich von 20-180°C die für die Veresterung erforderliche Menge an Polyol und gegebenenfalls Wasser zugibt, sodann die Veresterungsreaktion im wesentlichen vollständig ablaufen läßt und danach das Produkt gewinnt und schließlich das durch Umsetzung dieses Produktes mit der organischen Stickstoffbase erhaltene Salz oder Addukt mit wenigstens einem Aldehyd polykondensiert.

9. Verfahren nach Anspruch 8, **dadurch gekennzeichnet, daß** man im wesentlichen stöchiometrische Mengen an Phosphorpentoxid, Polyol und gegebenenfalls Wasser zugibt.

10. Verfahren nach Anspruch 8 oder 9, **dadurch gekennzeichnet, daß** man Polyol und gegebenenfalls Wasser bei einer Temperatur im Bereich von 20-150°C zugibt.

11. Verfahren nach einem der Ansprüche 8 bis 10, **dadurch gekennzeichnet, daß** man die Veresterungsreaktion bei wenigstens der Temperatur der Zugabe von Polyol und gegebenenfalls Wasser während einer Zeitdauer von 1-6 Stunden vervollständigt.

12. Verwendung eines Derivates nach einem der Ansprüche 1 bis 7 als flammhemmende und/oder intumeszierende Ausrüstung in Kunststofformlingen, auf porösen Trägern oder in Anstrich- bzw. Beschichtungssystemen.

13. Verwendung nach Anspruch 12 als Flammschutzmittel für Polyolefine.

## Claims

1. A derivative of phosphoric acid ester salt or adduct or mixtures thereof obtained from a reaction product of at least one polyol phosphoric acid ester with at least one organic nitrogen base **characterised in that** said reaction product is polycondensed with at least one aldehyde.

2. A derivative according to claim 1 **characterised in that** the reaction product is polycondensed with a C₁-C₄-monoaldehyde or a C₂-C₄-dialdehyde.

3. A derivative according to claim 1 or claim 2 **characterised in that** the reaction product is condensed with formaldehyde, acetylaldehyde or glyoxal.

4. A derivative according to one of claims 1 to 3 **characterised in that** it is polycondensed with 0.5-8, preferably 0.5-4, in particular 0.5-2 moles of monoaldehyde or with 0.25-4, preferably 0.25-2, in particular 0.25-1 mole of dialdehyde per mole of organic nitrogen base, preferably melamine.

5. A derivative according to one of claims 1 to 4 **characterised in that** the reaction product was obtained by reaction of 2,3-moles of organic nitrogen base per mole of polyol phosphoric acid ester.

6. A derivative according to one of claims 1 to 5 **characterised in that** the reaction product was obtained using melamine, guanidine or ethylene diamine, in particular melamine, as the nitrogen base.

7. A derivative according to one of claims 1 to 6 **characterised in that** the polyol phosphoric acid ester was obtained using at least one C₄-C₆-polyol, preferably pentaerythritol or sobitol.

8. A process for the production of a derivative of phosphoric acid ester salt or adduct or a mixture thereof by reacting at least one polyol with phosphorus pentoxide and reacting the polyol phosphoric acid ester obtained in that way with at least one organic nitrogen base forming a phosphoric acid ester salt or adduct, **characterised in that** the amount of phosphorus pentoxide required for the esterification operation is suspended in an amount of the ester or ester mixture to be produced, then at a temperature in the range of 20-180°C the amount of polyol required for the esterification operation and optionally water is added, then the esterification reaction is allowed to take place substantially completely and thereafter the product is obtained and finally the salt or adduct obtained by reaction of that product with the organic nitrogen base is polycondensed with at least one aldehyde.

9. A process according to claim 8 **characterised in that** substantially stoichiometric amounts of phosphorus pentoxide, polyol and optionally water are added.

10. A process according to claim 8 or claim 9 **characterised in that** polyol and optionally water are added at a temperature in the range of 20-150°C.

11. A process according to one of claims 8 to 10 **characterised in that** the esterification reaction is completed at at least the temperature of addition of polyol and optionally water during a period of 1-6 hours.

12. Use of a derivative according to one of claims 1 to 7 as a flame-inhibiting and/or intumescent treatment in plastic mouldings, on porous carriers or in paint or coating systems.

13. Use according to claim 12 as a flameproofing agent for polyolefins.

## Revendications

1. Dérivé d'un sel ou d'un adduit d'ester d'acide phosphorique, ou de mélanges de ceux-ci, obtenu à partir d'un produit de la réaction d'au moins un ester de l'acide phosphorique d'un polyol et d'au moins une base azotée organique, **caractérisé en ce que** ce produit de réaction est polycondensé avec au moins un aldéhyde.

2. Dérivé selon la revendication 1, **caractérisé en ce que** le produit de réaction est polycondensé avec un monoaldéhyde en C₁-C₄ ou un dialdéhyde en C₂-C₄.

3. Dérivé selon la revendication 1 ou 2, **caractérisé en ce que** le produit de réaction est polycondensé avec du formaldéhyde, de l'acétaldéhyde ou du glyoxal.

4. Dérivé selon l'une des revendications 1 à 3, **caractérisé en ce qu'**il est polycondensé avec 0,5 à 8, de préférence 0,5 à 4 et en particulier 0,5 à 2 moles d'un monoaldéhyde ou avec 0,25 à 4, de préférence 0,25 à 2 et en particulier de 0,25 à 1 mole de dialdéhyde par mole de base azotée organique.

5. Dérivé selon l'une des revendications 1 à 4, **caractérisé en ce que** le produit de réaction a été obtenu par réaction de 2 à 3 moles d'une base azotée organique par mole d'un ester d'acide phosphorique d'un polyol.

6. Dérivé selon l'une des revendications 1 à 5, **caractérisé en ce que** le produit de réaction a été obtenu par utilisation, en tant que base azotée, de mélamine, de guanidine ou d'éthylènediamine, en particulier de mélamine.

7. Dérivé selon l'une des revendications 1 à 6, **caractérisé en ce que** l'ester d'acide phosphorique d'un polyol a été obtenu par utilisation d'au moins un polyol en C₄-C₆, de préférence le pentaérythritol ou le sorbitol.

8. Procédé de préparation d'un dérivé d'un sel ou adduit d'un ester d'acide phosphorique ou d'un mélange de ceux-ci par réaction d'au moins un polyol avec du pentoxyde de phosphore et réaction de l'ester d'acide phosphorique d'un polyol ainsi obtenu avec au moins une base azotée organique, avec formation d'un sel ou d'un adduit d'un ester d'acide phosphorique, **caractérisé en ce que** l'on met en suspension la quantité nécessaire à l'estérification du pentoxyde de phosphore dans une certaine quantité de l'ester ou du mélange d'esters à préparer, puis, à une température comprise dans la plage de 20 à 180°C, on ajoute la quantité de polyol, et éventuellement d'eau, nécessaire à l'estérification, puis on assure un déroutement pour ainsi dire complet de la réaction d'estérification, puis on récupère le produit, et finalement on soumet à une polycondensation, avec au moins un aldéhyde, le sel ou adduit obtenu par réaction de ce produit avec la base azotée organique.

9. Procédé selon la revendication 8, **caractérisé en ce que** l'on ajoute des quantités pour ainsi dire stoechiométriques de pentoxyde de phosphore, de polyol et éventuellement d'eau.

10. Procédé selon la revendication 8 ou 9, **caractérisé en ce qu'**on ajoute le polyol et éventuellement l'eau à une température comprise dans la plage de 20 à 150°C.

11. Procédé selon l'une des revendications 8 à 10, **caractérisé en ce qu'**on parachève la réaction d'estérification au moins à la température d'addition du polyol et éventuellement de l'eau, pendant un laps de temps de 1 à 6 heures.

12. Utilisation d'un dérivé selon l'une des revendications 1 à 7, en tant qu'additif inhibiteur de flamme et/ou intumescent dans des ébauches plastiques, sur des supports poreux ou dans des systèmes de peinture ou de revêtement.

13. Utilisation selon la revendication 12 en tant qu'agent d'ignifugation de polyoléfines.
